# EUROPEAN PATENT SPECIFICATION

(11) **EP 1 272 043 B1**
(45) Date of publication and mention of the grant of the patent: **30.08.2006**
(21) Application number: 01919501.5
(22) Date of filing: 14.03.2001
(51) Int. Cl.: A23C 7/04

(54) **LACTAMASE COMPOSITION FOR TREATMENT OF MILK**
LACTAMASEZUSAMMENSETZUNG ZUR BEHANDLUNG VON MILCH
COMPOSITIONS DE LACTAMASE DESTINEES AU TRAITEMENT DU LAIT

(30) Priority: 14.03.2000 FI 20000112 U
(43) Date of publication of application: 08.01.2003
(73) Proprietor: Finnzymes Oy, 02201 Espoo (FI); Vetcare Oy, 24101 Salo (FI)
(72) Inventor: MAJAHARJU, Jukka, FIN-02770 Espoo (FI); TENKANEN, Tuomas, FIN-02360 Espoo (FI); HEINONEN, Kalevi, FIN-04840 Hautjärvi (FI)
(74) Representative: Sundman, Patrik Christoffer
(86) International application number: PCT/FI2001/000253
(87) International publication number: WO 2001/067879

(56) References cited:
- FI-A- 971 902
- FI-U- 4 533
- M. KORYCKA-DAHL ET AL.: 'Use of microbial beta-lactamase to destroy penicillin added to milk' J. DAIRY SCI. vol. 68, 1985, pages 1910 - 1916, XP002962033

## Description

The present invention relates to the enzyme composition according to the preamble of Claim 1 for the treatment of the milk of a cow treated with antibiotics.

The ability of the β-lactamase enzyme to degrade β-lactam antibiotic residues in, for example, milk has been known since the 1950s. It has been considered in general that milk containing β-lactamase residues cannot be used, for example, as human food. This is due to the fact that all β-lactamase residues in milk cannot be inactivated by means of heat; even after treatment at 100 °C nearly 40 % of the enzyme activity remains in the milk. The intake of such milk products may even be dangerous to individuals receiving oral penicillin treatment. Thus Korycka-Dahl *et al.* are of the opinion that products treated with lactamase should be provided with warning notations (Korycka-Dahl *et al.,* Journal of Dairy Science 68 (1985) 1910-1916).

FI patent application 971902 describes an option of a new type for the treatment and exploitation of antibiotic-containing milk. The said invention is based on the idea that milk treated with β-lactamase enzyme is given as such to animals as fodder or is used as raw material for fodder. By means of this option, milk otherwise to be regarded almost as a problem waste can be exploited usefully.

In their above-mentioned article Korycka-Dahl *et aL* used a raw lactamase preparation supplied by Sigma Chemical Co. In order to take into consideration the safety in use of products treated with lactamase, the article proposes that the conventional enzyme be replaced with an immobilized enzyme. In FI patent application 971902, on the other hand, the enzyme treatment is carried out with a purified β-lactamase produced by a *Bacillus cereus* bacterial strain, the lactamase being completely free of bacteria capable of propagation.

Known enzyme preparations are mainly developed for laboratory use and are not as such suited for the treatment of very large batches of milk in practical conditions, for example, in cow barns. Also, they have not been formulated, for example, for the method of FI patent application 971902.

The object of the present invention is to provide a commercial β-lactamase composition with which milk can be treated safely and which is suitable for use in particular in those cases in which it is desired to feed the enzyme-treated milk as fodder to animals.

The invention is based on the idea that the β-lactamase preparation contains, in addition to the enzyme, enzyme-stabilizing components, a buffer and a stabilizing agent, as well as a colorant or some other indicator. The activity (amount) of β-lactamase in the composition must be suitable for the treatment of product batches of tens or even a few hundred liters in practical conditions, without the amount of unreacted lactamase residues (and the amount of other components of the preparation) in the milk becoming too large. On the other hand, the activity of the product must be sufficient so that no β-lactam residues are left in the treated milk. Thus the activity is set at 100 - 10,000 U/liter of milk. By means of the buffer the pH value of the enzyme preparation is set within the optimum range required by practical applications, in particular the use mentioned above. The buffer for its part stabilizes the enzyme, and with a separate stabilizing agent the precipitation of the enzyme during cold storage is prevented. It is further essential in terms of the invention that there is added to the enzyme preparation a colorant capable of coloring the product treated with the enzyme preparation so that the treated batch of milk can be distinguished from untreated milk.

On the basis of what has been stated above, the β-lactamase composition according to the invention generally contains the following components:
- β-lactamase having an activity (amount) of approximately 100 - 10,000 U/liter of milk to be treated, in which case the composition generally contains approximately 10,000
- 100,000 U/ml,
- a sufficient amount of buffer to buffer the pH value of the composition to 6 - 8,
- a stabilizing agent for preventing the inactivation and/or precipitation of β-lactamase during cold storage of the composition, and
- a colorant for the identification of the product treated with the composition.

More precisely, the enzyme composition according to the invention is characterized in what is stated in the characterizing part of Claim 1.

The invention provides considerable advantages. Thus, by means of the invention, in cow barn conditions, it is possible without technical equipment to remove penicillin and certain other β-lactam antibiotics from milk and other fodders. Owing to its enzyme activity the enzyme preparation is suitable for use for the treatment of batches of a few tens to a few hundreds of liters of milk. If the volume of the enzyme preparation is suitably selected, it can be used for treating raw milk batches of up to tens of thousands liters (e.g. 30,0001). The enzyme-treated milk or corresponding product can be given, for example, to calves as a drinkable fodder, or it can be used as a raw material for fodder (for example, milk powder can be prepared from it). According to a preferred embodiment, the indicator used is a colorant for coloring the product treated with the composition (cf. below in greater detail), in which case milk treated with one vial of the product (volume, for example, 1 ml), intended for a milk batch of 40 liters, is colored, for example, light blue still at a volume of approximately 240 1, in which case the color is still visually observable and the milk can be distinguished from untreated milk.

By the use of the preparation now developed it is also possible to remove β-lactam antibiotics other than penicillin, for example, ampicillin and amoxycillin. By a widening of the specificity of the enzyme it is possible also to remove antibiotics partly resistant to β-lactamase, for example, cephalosporins. The preparation can be used for treating not only cow's milk but also the milk of other mammals, such as goat's milk.

The invention will be described below in greater detail with the help of a detailed description.

The active component used in the enzyme composition is a purified β-lactamase enzyme, preferably that of *Bacillus licheniformis,* produced in a *Bacillus subtilis* strain. The β-lactamase activity (amount) is approximately 100 - 10,000 U/liter of milk. In liquid form the enzyme composition therefore contains approximately 10,000 - 100,000 U/ml, most preferably approximately 15,000 - 50,000 U/ml, typically approximately 20,000 - 30,000 U/ml. The amount is such that the antibiotic residues in milk will decrease to the MRL (Maximum Residue Limit) level or below it. In the example case below, the lactamase activity of a product vial of 1 ml was 24,000 U, with which amount 401 of milk can be treated, in which case 600 U of lactamase is used for the treatment of one liter of milk.

The activity units indicated above mean the enzyme amounts obtained using nitrocefin as the substrate for lactamase (O'Callaghan, C.H., Morris, A., Kirby, S., Shingler, A.H., 1972 Antimicrobial Agents and Chemotherapy, Vol. 1, 282 - 288). The activity can also be measured by other methods, but in that case it would also be necessary to determine the correlation with the nitrocefin method.

As was pointed out above, the preparation contains the enzyme in a buffer solution. The enzyme is preferably completely or at least partly dissolved in the solution, since this promotes the use of the preparation. The solution contains a buffering component, at least one stabilizing agent, and a colorant.

The buffer used is, for example, a phosphate compound, such as potassium or sodium phosphate, the function of which is to stabilize, activate and protect the lactamase. By means of the buffer the pH can be set at a value of 6 - 8, typically approximately 7.0, which is suitable in terms of the preservation and action of the lactamase enzyme. The concentration of the phosphate buffer may vary within the range 1 mM - 100 mM. An example that can be mentioned of a suitable calcium phosphate buffer is a K₂HPO₄ - KH₂PO₄ buffer. This may, however, be replaced with some other non-toxic buffer or another buffer suitable for use in fodder.

In addition to the buffer there is incorporated into the enzyme preparation a stabilizing agent the function of which is to stabilize the preparation so that the lactamase will not lose its activity even during a long storage. At the same time it is a function of the stabilizing agent to prevent the precipitation of the enzyme during many freezing-melting cycles. It is necessary that the product can be stored both in a freezer and in a refrigerator. The latter is important, since in a cow barn freezer storage is not always possible. Preferably the stabilizing agent used is a polyol, such as glycerol, or an amino acid based preparation, such as gelatin. Other suitable stabilizing agents include ion-free detergents, such as polyozyethylene-based detergents, e.g. polyoxyethylene sorbitane mono-oleate (polysorbate 80), polyoxyethylene sorbitane monolaurate (polysorbate 20), polyoxyethylene lauryl ethyl (laureth 4), and polyoxyethylene, polyoxypropylene block polymer (poloxamer 188).

Glycerol is regarded as especially advantageous, since its cold properties are suitable. Other polyols are also suitable. Glycerol may be added in an amount of at least 10 % of the volume, preferably the preparation contains glycerol approximately 20 - 60 %, typically approximately one half (50 %). This glycerol amount prevents the product from freezing at -20°C.

The concentrations of the other stabilizing agents vary within wide limits. Thus, gelatin can be used at approximately 0.1 - 4 % of the volume of the preparation. The amounts used of ion-free detergents are approximately 1 - 20 % by volume.

In addition to a buffer and a cold-storage stabilizing agent, the lactamase preparation may contain other components stabilizing the preparation. It is especially preferable to incorporate a preservative that inhibits any bacterial growth during storage. Benzoates, such as alkylparahydroxybenzoates, can be used as such a preservative. Methylparahydroxybenzoate and propylparahydroxybenzoate can be mentioned as advantageous examples. When glycerol is used as the principal stabilizing agent, the preservative can be combined with the preparation with the help of an auxiliary solvent, in particular an organic auxiliary solvent. The auxiliary solvent used is, for example, ethanol.

The concentration of a benzoate preservative in the enzyme preparation is in general 0.01 - 100 mg/ml, preferably approximately 0.05 - 50 mg/ml. The minimum concentration of methylparahydroxybenzoate is approximately 1.4 mg/ml and that of propylparahydroxybenzoate approximately 0.2 mg/ml.

According to a preferred embodiment of the invention, the product further contains a colorant so that the user of the product can distinguish a treated milk batch from an untreated batch, and in order to prevent a treated milk batch from ending up in dairy use. The colorant used is a food color capable of coloring the entire milk batch even after dilution (for example, if the treated milk batch is accidentally combined with an untreated batch). A food color that has been given an E-code is usable as the colorant.

In general the colorant should color a milk batch of at least 2001. The colorant must be water-soluble, and preferably such that its color is stable, in particular at room temperature and when not protected from light. Furthermore, it is advantageous if the color of the colorant will not fade or change significantly when the milk becomes sour. Blue and red are preferable colors, since they are the easiest to distinguish visually.

Examples that can be mentioned of especially preferable colors include E133 (Brilliant Blue FCF), E131 (Patent Blue), E120 (Carmine Red), and E132 (Indigo Carmine). Color E133 has excellent coloring capability and it retains its color even when stored at room temperature.

Color E133 in amounts of 50 - 100 mg is capable of coloring milk light blue at a volume of 240 of milk, in which case the treated milk batch is still identifiable visually.

In a preparation that contains glycerol as a stabilizing agent, the concentration of the E133 colorant may be approximately 10 - 200 mg, e.g. approximately 84 mg, per volume (ml) of the enzyme composition. The treated milk is used for fodder, not for human food. The upper limit for the colorant concentration is determined according to the viscosity of the preparation and the dosing device used. For example, when conventional pipettes are used for the dosing, the concentration of the E133 colorant cannot be raised much higher than 200 mg/ml, since the viscosity of the product becomes too high. The concentration limits for other colorants vary according to the colorant, but they can be easily determined experimentally.

In addition to food colors it is possible to use, for example, azo colors.

All of the components mentioned above have been selected so that their grade corresponds at least to the food grade.

The enzyme preparation according to the invention is liquid, and the enzyme and colorant or other indicator therein is in general in a dissolved state. The solution is packed in unit packages of the desired size, for example, in glass or plastic bottles or vials, which are closed tightly.

As an advantageous example there can be mentioned an enzyme preparation having the following composition (a product vial of 1 ml):
β-lactamase 20,000 - 30,000 U,
potassium phosphate buffer (K₂HPO₄-KH₂PO₄) 1-100 mM,
glycerol 20 - 60 %,
alkylparahydroxybenzoate 0.05 - 50 mg/ml, and
E133 color 50 - 150 mg/ml.

The preparation described above removes antibiotic residues from milk during penicillin treatment and the subsequent precautionary period.

### Example

An enzyme preparation having the following composition was prepared:
β-lactamase (Antipen) 24,000 U
K₂HPO₄-KH₂PO₄, pH 7.0 10 mM
Glycerol 50 %
Methylparahydroxybenzoate 1.4 mg/ml
Ethanol 0.7 % (vol./vol.)
E133 color 84 mg/ml

Total content: 1 ml. The storage temperature recommendation for the product is -20 °C, or also refrigerator temperature (+6 °C). The product is capable of coloring at least 2401 light blue.

## Claims

1. A β-lactamase composition intended for the treatment of milk containing
- β-lactamase the activity of which, determined on a nitrocefin substrate, is approximately 100 - 10,000 U/liter of milk to be treated,
- a buffer in a sufficient amount to buffer the pH value of the composition to 6 - 8,
- a stabilizing agent for inhibiting the inactivation and/or precipitation of β-lactamase during cold storage of the composition, and
- a colorant capable of coloring the treated milk for identifying the milk treated with the composition.

2. The composition according to Claim 1, **characterized in that** the pH of the composition is approximately 7.0.

3. The composition according to Claim 1 or 2, **characterized in that** the buffer is potassium phosphate or sodium phosphate.

4. The composition according to any of Claims 1-3, **characterized in that** the stabilizing agent is glycerol or gelatin.

5. The composition according to Claim 4, **characterized in that** the amount of glycerol is approximately 20 - 60 % by weight of the total amount of the composition.

6. The composition according to any of the preceding claims, **characterized in that** it contains a preservative that inhibits bacterial growth during storage.

7. The composition according to Claim 6, **characterized in that** the preservative is a benzoate, such as methylparahydroxybenzoate or propylparahydroxybenzoate.

8. The composition according to any of the preceding claims, **characterized in that** it contains a food color in a sufficient concentration to color a milk batch of at least 2001.

9. The composition according to Claim 8, **characterized in that** the colorant is E133.

10. The composition according to any of the preceding claims, **characterized in that** it contains in a product vial of 1 ml
β-lactamase 20,000-30,000 U,
K₂HPO₄-KH₂PO₄ 1-100 mM,
Glycerol 40-60%
alkylparahydroxybenzoate 0.05 - 50 mg/ml, and
E133 color 50-150 mg/ml.

## Patentansprüche

1. Eine β-Lactamase Zusammensetzung, die für die Behandlung von Milch gedacht ist, die
- β-Lactamase, dessen Aktivität, die auf einem Nitrocefin-Substrat bestimmt wird, bei etwa 100-10.000 U/Liter zu behandelnder Milch liegt,
- ein Puffer in einer ausreichenden Menge, um den pH-Wert der Zusammensetzung auf 6-8 zu puffern,
- ein Stabilisierungsagens zum Inhibieren der Inaktivierung und/oder Präzipitierung von β-Lactamase während der Kühllagerung der Zusammensetzung, und
- ein Farbmittel, das die behandelte Milch zum Identifizieren der Milch, die mit der Zusammensetzung behandelt wurde, anfärbt, enthält.

2. Die Zusammensetzung gemäß Anspruch 1, **dadurch gekennzeichnet, dass** der pH der Zusammensetzung bei etwa 7,0 liegt.

3. Die Zusammensetzung gemäß Anspruch 1 oder 2, **dadurch gekennzeichnet, dass** der Puffer Kaliumphosphat oder Natriumphosphat ist.

4. Die Zusammensetzung gemäß einem der Ansprüche 1-3, **dadurch gekennzeichnet, dass** das Stabilisierungsagenz Glycerol oder Gelatine ist.

5. Die Zusammensetzung gemäß Anspruch 4, **dadurch gekennzeichnet, dass** die Menge des Glycerols bei etwa 20-60% des Gewichts der Gesamtmenge der Zusammensetzung liegt.

6. Die Zusammensetzung gemäß einem der vorhergehenden Ansprüche, **dadurch gekennzeichnet, dass** sie ein Konservierungsmittel enthält, das das bakterielle Wachstum während der Lagerung inhibiert.

7. Die Zusammensetzung gemäß Anspruch 6, **dadurch gekennzeichnet, dass** das Konservierungsmittel ein Benzoat, wie zum Beispiel Methylparahydroxybenzoat oder Propylparahydroxybenzoat, ist.

8. Die Zusammensetzung gemäß einem der vorhergehenden Ansprüche, **dadurch gekennzeichnet, dass** sie eine Lebensmittelfarbe in einer ausreichenden Konzentration enthält, um eine Milchmenge von wenigstens 200 1 anzufärben.

9. Die Zusammensetzung gemäß Anspruch 8, **dadurch gekennzeichnet, dass** das Farbmittel E133 ist.

10. Die Zusammensetzung gemäß irgendeinem der vorhergehenden Ansprüche, **dadurch gekennzeichnet, dass** sie in einer Produktampulle von 1 ml
β-Lactamase 20.000-30.000 U,
K₂HPO₄-KH₂PO₄ 1-100 mM,
Glycerol 40-60 %,
Alkylparahydroxybenzoat 0,05-50 mg/ml, und
E133 Farbe 50-150 mg/ml enthält.

## Revendications

1. Composition de β-lactamase destinée au traitement du lait contenant :
- de la β-lactamase dont l'activité, déterminée sur un substrat de nitrocéfine, est d'environ 100 à 10 000 u/litre de lait à traiter,
- un tampon en une quantité suffisante pour tamponner la valeur pH des compositions à 6-8.
- un agent stabilisateur pour inhiber l'inactivation et/ou la précipitation de la β-lactamase pendant l'entreposage frigorifique de la composition, et,
- un colorant capable de colorer le lait traité pour identifier le lait traité avec la composition.

2. Composition selon la revendication 1, **caractérisée en ce que** le pH de la composition est d'environ 7,0.

3. Composition selon la revendication 1 ou 2, **caractérisée en ce que** le tampon est du phosphate de potassium ou du phosphate de sodium.

4. Composition selon l'une quelconque des revendications 1 à 3, **caractérisée en ce que** l'agent stabilisateur est le glycérol ou la gélatine.

5. Composition selon la revendication 4,
**caractérisée en ce que** la quantité de glycérol est d'environ 20 à 60 % en poids de la quantité totale de la composition.

6. Composition selon l'une quelconque des revendications précédentes, **caractérisée en ce qu'**elle contient un conservateur qui inhibe la croissance bactérienne pendant le stockage.

7. Composition selon la revendication 6, **caractérisée en ce que** le conservateur est un benzoate, tel que le méthylparahydroxybenzoate ou le propylparahydroxybenzoate.

8. Composition selon l'une quelconque des revendications précédentes, **caractérisée en ce qu'**elle contient un colorant alimentaire en une concentration suffisante pour colorer un lot de lait d'au moins 200 1.

9. Composition selon la revendication 8,
**caractérisée en ce que** le colorant est E133.

10. Composition selon l'une quelconque des revendications précédentes, **caractérisée en ce qu'**elle contient dans une fiole de 1 ml :
β-lactamase 20 000 à 30 000 U,
K₂HPO₄-KH₂PO₄ 1 à 100 mM,
glycérol 40 à 60 %,
alkylparahydroxybenzoate 0,05 à 50 mg/ml, et colorant E133 50 à 150 mg/ml.
